# EUROPEAN PATENT APPLICATION

(11) **EP 0 743 310 A1**
(43) Date of publication of application: **20.11.1996**
(21) Application number: 96302347.8
(22) Date of filing: 02.04.1996
(51) Int. Cl.: C07D 263/06, C07D 265/06, C11D 1/52

(54) **Surfactants and compositions containing them**

(30) Priority: 18.05.1995 US 444335; 18.05.1995 US 444333
(71) Applicant: UNILEVER PLC, London EC4P 4BQ (GB); UNILEVER N.V., 3013 AL Rotterdam (NL)
(72) Inventor: Rahman, Mohammed Abdur, Columbus, Ohio 43235 (US); Wu, Shang-Ren, Mahwah, New Jersey 07430 (US); Hung, Anthony, New City, New York 10956 (US)
(74) Representative: Fransella, Mary Evelyn

(57) **Abstract**

Oxazolidine amides (5-membered ring) and tetrahydrooxazine amides (6-membered ring) are novel, biodegradable, sugar-based surfactants, useful in detergent, cleaning and personal care compositions.

## Description

### TECHNICAL AREA

The present invention relates to novel oxazolidine amide (5-membered ring) and tetrahydrooxazine amide (6-membered ring) surfactants, to methods for preparing the surfactants, and to detergent and personal product compositions containing them. These compounds are biodegradable, sugar-based surfactants.

### BACKGROUND AND PRIOR ART

It has in recent years become a highly desirable goal in the art to find surfactants which are environmentally friendly and preferably not tremendously expensive. Carbohydrate based surfactants are good candidates in this regard because they offer the possibility of cheap, renewable and biodegradable surfactants.

Several carbohydrate based amide surfactants are known in the art.

In US 5 389 279 (Au et al), for example, there are taught certain aldobionamide compounds. These compounds are structurally different from the compounds of the subject invention.

US 5 009 814 (Kelkenberg et al) provides N-polyhydroxyalkyl fatty acid amides used as thickeners in aqueous surfactant systems and having the formula: 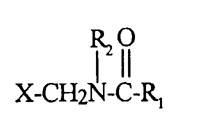 wherein R₁ is alkyl, R₂ is hydrogen, alkyl or hydroxy alkyl and X is a polyhydroxy group.

A series of Procter and Gamble patent applications teaches various compositions which comprise polyhydroxy amides. WO 92 06172A, for example, teaches built liquid detergent compositions containing polyhydroxy fatty acid amides.

The polyhydroxy fatty acid amides are generally linear structures (ie wherein the polyhydroxy group is derived from monosaccharides such as in the case of N-methyl glucamide). Such linear structures would be expected to have strong intermolecular interaction lelading to, for example, higher Krafft points and therefore to be less soluble than cyclic surfactants such as the compounds of the invention (Krafft point is a measure of solubility; specifically, it is the temperature at which the solubility of the nonionic surfactant becomes equal to its critical micelle concentration). Even if the polyhydroxy amide is a disaccharide, the compounds still have an extended linear structure within the molecule which differs from the compounds of the invention.

Polyhydroxy fatty acid amides with a reverse amide link from the polyhydroxy fatty amides noted above (eg N-alkyl gluconamides of general structure HOCH₂(CHOH)₄CONHR) are also known in the art, for example, in US 2 662 073 (Mehltretter et al). As noted, these are either linear structures which would be expected to have higher Krafft points (ie be less soluble) than cyclic compounds; or they have extended linear structures within the molecules which would also be expected to raise the Krafft point.

Thus, it would be advantageous to find a carbohydrate based surfactant with a structure providing greater solubility. In addition, it is always desirable to find a novel carbohydrate surfactant, whether or not it has a cyclic structure.

### DEFINITION OF THE INVENTION

The present invention provides a compound of the formula I, ie having one of the formulae I(a) or I(b): 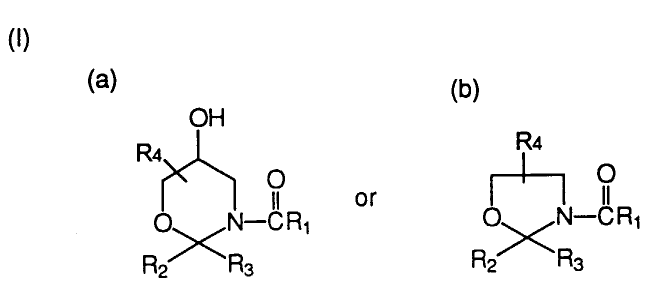 wherein:
R₁ is a linear or branched alkyl or alkenyl group having from 1 to 50, preferably 1 to 40 and more preferably from 8 to 24 carbon atoms;
each of R₂ and R₃, which may be the same or different, is hydrogen or a linear or branched alkyl or alkenyl group having from 1 to 50, preferably from 1 to 40 and more preferably from 8 to 24 carbon atoms; and
R₄ represents the residue of a reducing sugar or an amino sugar.

The invention further provides a process for the preparation of a compound of the formula I, which process comprises:
(i) cyclising an amino sugar to form either a 5-membered oxazolidine or 6-membered tetrahydrooxazine,
(ii) amidating the 5-membered oxazolidine or 6-membered tetrahydrooxazine to form a compound of the formula I.

The invention further provides a detergent, cleansing or personal care composition comprising a surfactant system which consists wholly or partially of a compound of the formula I.

### DETAILED DESCRIPTION OF THE INVENTION

### The compounds

The compounds of the formula I are ultimately derived from a reducing sugar, converted by reductive amination to an amino sugar (eg glucamine or glucosamine), which on cyclisation gives a 5-membered (oxazolidine) or 6-membered (tetrahydrooxazine) ring compound which on amidation gives a compound of the formula I.

The structure of R₄ will therefore be determined by the structure of the original reducing sugar prior to the reductive amination to form the intermediate amino sugar.

It should be noted from the formulae above that the R₄ group may be attached at varying places in the ring depending on the starting reducing sugar or amino sugar.

R₄, for example, may be hydrogen in the case of the 6-membered ring or CH₂OH in the case of the 5-membered ring when the starting sugar is glyceraldehyde.

Suitable reducing sugars (starting sugars) which will define R₄ include glucose, fructose, maltose, lactose, galactose, mannose, xylose, erythritose and as noted above, glyceraldehyde. Starting sugars could also be the amino sugars such as glucamine or glucosamine. As raw materials, high dextrose corn syrup, high fructose corn syrup, and high maltose corn syrup can be utilized as well as the individual sugars listed above. These corn syrups may yield a mix of sugar components for use in yielding the final R₄. It should be understood that it is by no means intended to exclude other suitable raw materials.

R₄ preferably will be selected from the group consisting of -(CHOH)ₙ -CH₂OH where n is an integer from 1 to 5, inclusive. Most preferred depends on whether the resulting compound is the five or six member ring compound. In the five member ring, n = 3 and, in the six member ring, n = 2. Again, depending on the starting sugar or amino sugar, R₄ can be polysaccharide or residual polysaccharide structure.

In a specific embodiment of the invention, the compound has one of the following structures: 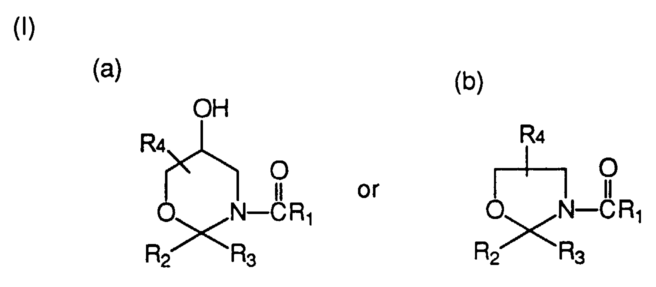 wherein R₄ = -(CHOH)ₙ,- CH₂OH, where, when it is structure (b), n' equals 3; and, when it is structure (a), n' equals 2; and R₁, R₂ and R₃ are as defined as in the formula (I) above.

Other preferred embodiments of the invention include, but are not limited to
(1) R₂ = R₃ = hydrogen; and R₁ = C₁₁ to C₁₇; and
(2) R₂ = hydrogen; R₁ = CH₃; and R₃ = C₁₁ to C₁₇.

It is highly preferred that the combination of R₁ and R₂ and R₃ should equal C₈ or greater, preferably C₈ to C₅₀, more preferably C₁₂ to C₃₀.

In one embodiment of the invention, the compound has the following structure: 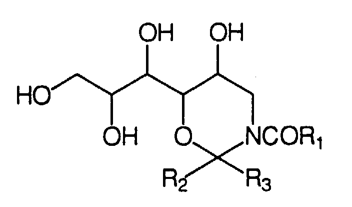 wherein
R₁ = a C₁ to C₅₀ alkyl or alkenyl group as defined above; and
R₂, R₃ = H or C₁ to C₅₀ alkyl or alkenyl group as defined above.
R₁ plus R₂ plus R₃ are preferably at least C₈, preferably C₁₂ to C₃₀, more preferably C₁₂ to C₂₄.

In a preferred embodiment of the invention, R₂ and R₃ are hydrogen or C₁ to C₄ alkyl and R₁ is a C₈ to C₂₄ straight chain alkyl group.

In another preferred embodiment, either R₂ and R₃ is C₈ to C₂₄ alkyl and R₁ is a C₁ to C₆ short chain alkyl group. While not wishing to be bound by theory, it is believed that enhanced surfactancy properties will be realized if, when either one of R₁, R₂ or R₃ is long chained, then the others are long chained (ie only one long chain is required).

In another preferred embodiment the compound has the following structure: 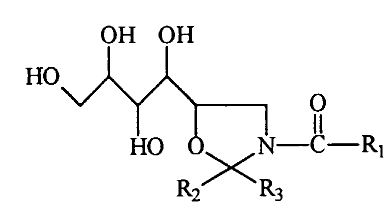 wherein
R₁ = C₁ to C₅₀ alkyl or alkenyl as defined above;
R₂, R₃ = H or C₁ to C₅₀ alkyl or alkenyl as defined above;
R₁ plus R₂ plus R₃ are preferably at least C₈,
preferably C₁₂ to C₃₀, more preferably C₁₂ to C₂₄

Preferably:
R₁ = C₁ to C₄ straight chain alkyl; and
R₂ or R₃ is C₈ to C₂₄ straight chain alkyl.

Especially preferred compounds of the invention are C₁₂ and C₁₄ oxazolidine acetamides, and C₁₂ tetrahydrooxazine myristamide.

### Process for preparing the compounds

As explained above the compounds of the invention may be prepared by amidation of the corresponding oxazolidine (5-membered ring) or tetrahydrooxazine (6-membered ring), which in turn may be prepared by cyclisation of an amino sugar. Amino sugars may be prepared by reductive amination of a sugar. The sugar from which the amino sugar is derived will determine the group R₄ in the compound of the invention.

Amidation may be effected by reacting the cyclic amine with a acid anhydride or acyl halide (preferably acyl chloride).

Cyclisation of the amino sugar may be effected by reaction with an aliphatic aldehyde. The reaction conditions (time, temperature, catalyst, solvent etc) depend on the chain length of the aldehyde.

The choice of aldehyde and the choice of anhydride (or acyl chloride) determine the length of R₂, R₃ and R₁, respectively. These are generally chosen such that, wherein R₁ is short-chained (eg C₁ to C₆), R₂ and/or R₃ will be long-chained (eg C₈ to C₄₀, preferably C₁₂ to C₃₀, more preferably C₁₂ to C₂₄; and when R₁ is long-chained (C₈ to C₄₀), R₂ and/or R₃ are hydrogen or short chain alkyl. While not wishing to be bound by theory, this is believed to be desirable in terms of optimising the surfactancy of the molecule. R₁ plus R₂ plus R₃ should preferably be at least C₈ , more preferably at least C₁₂.

When the aldehyde has a chain length of 8 to 30 carbon atoms the reaction may be carried out in the presence of solvent at reflux temperature and requires 12 to 24 hours. Amidation is carried out at a lower temperature. One process according to the invention therefore comprises:
(i) reacting the amino sugar with a C₈-C₃₀ aldehyde in the presence of a solvent and acid catalyst at a reflux temperature for 12 to 24 hours,
(ii) cooling the reaction mixture to a temperature within the range of from 5 to 25°C and adding an acid anhydride or acid halide, and recovering the compound of the formula I.

Suitable solvents include anhydrous methanol, ethanol, propanol, isopropanol, ethylene glycol, propylene glycol, ethylene glycol monomethyl ether and diethylene glycol.

Suitable acid catalysts include sulphonic acids, such as p-toluenesulphonic acid, methanesulphonic acid or alkyl benzenesulphonic acid; and acid resins such as Amberlite (Trade Mark) IR - 120 ex Aldrich.

When the aldehyde has a chain length of 1 to 8 carbon atoms, the cyclisation reaction may be carried out at ambient temperature and requires 20 to 30 hours. The temperature is lowered to about 0°C for the amidation step. A second process of the invention therefore comprises
(i) reacting the amino sugar, dissolved in a solvent, with a C₁-C₈ aldehyde at ambient temperature for 20 to 30 hours,
(ii) cooling the reaction mixture to a temperature of from -5 to +5°C and adding an acid anhydride or acyl halide, and recovering the compound of the formula I.

Suitable solvents for step (i) include methanol, ethanol, propanol, isopropanol, ethylene glycol, propylene glycol, monomethyl ether and diethylene glycol.

### General Method for the Preparation of Oxazolidine Amides

The oxazolidine amides were synthesised by the reaction of available sugar amines, for example, glucamine or 1-amino-1-deoxysorbitol, with various long chain aldehydes. Glucamine may be synthesised by reductive amination of glucose by ammonia. The sugar amine (eg glucamine) was dissolved in a solvent such as anhydrous methanol and refluxed for 12 to 24 hours with stirring to form a clear solution. Other suitable solvents include ethanol, propanol, isopropanol, ethylene glycol, propylene glycol, ethylene glycol monomethyl ether and diethylene glycol. Equimolar amounts of fatty aldehyde were added and refluxed in solvent (eg anhydrous methanol) with an acid catalyst. Suitable catalyst include, but are not limited to sulphonic acids, such as p-toluenesulphonic acid, methanesulphonic acid or alkyl benzenesulphonic acid; and acid resins such as Amberlite (Trade Mark) IR-120 ex Aldrich.

The amidation step (in the same reaction vessel) involved cooling the reaction to about 10°C to 25°C with an ice bath and adding 1.0 to 1.5 equivalent of anhydride. Suitable anhydrides include any component of formula: 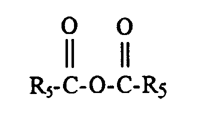 where R₅ is C₁ to C₃₀, preferably C₁ to C₄.

The solvent was removed under reduced pressure and the crude product purified by washing with, for example, hexane and recrystallisation in acetone or ethyl acetate.

### General Method for the Preparation of Tetrahydrooxazine Amides

Sugar amine (eg glucamine) was dissolved in refluxing solvent such as methanol for 2 to 4 hours with stirring until the solution turned clear. Other solvents which could be used include ethanol, propanol, isopropanol, ethylene glycol, propylene glycol, monomethyl ether and diethylene glycol. The solution was cooled to room temperature and an aldehyde (eg formaldehyde or short chain aldehyde) solution was added. The reaction was allowed to proceed for about 24 hours and then all the solvent was removed under reduced pressure. This syrupy intermediate was not purified and was used for the next step. This material was dissolved in a solvent system (eg 2:1 dimethylformamide:pyridine) and acylated with the appropriate long chain acid chloride (1.0 to 1.5 equivalents) at 0°C. The product was purified by extraction and recrystallised in the appropriate solvent.

### Surfactant use

The invention is also concerned with the use of the compounds of the invention as surface-active agents (surfactants). They may be used for this purpose in, for example, detergents for fabric washing, dishwashing and general purpose cleaning, and personal cleaning and care compositions such as soap bars, shampoos, shower gels, toothpastes and cosmetics.

The invention therefore also provides a detergent, cleaning or personal care composition comprising a surfactant system which consists wholly or partially of a compound of the invention.

The compound of the invention may suitably constitute from 0.01 to 60 wt%, preferably from 0.1 to 45 wt% and more preferably from 1 to 20 wt%, of the detergent, cleaning or personal care composition.

### Detergent compositions

The compounds of the present invention may be incorporated in detergent compositions of all physical types, for example, powders, liquids, gels and solid bars.

These compositions will generally contain other detergent-active compounds, in addition to the compounds of the invention, and detergency builders, and may optionally contain bleaching components and other active ingredients to enhance performance and properties.

The detergent compositions of the invention will contain, as essential ingredients, one or more detergent-active compounds (surfactants) which may be chosen from soap and non-soap anionic, cationic, nonionic, amphoteric and zwitterionic detergent-active compounds, and mixtures thereof. Many suitable detergent-active compounds are available and are fully described in the literature, for example, in "Surface-Active Agents and Detergents", Volumes I and II, by Schwartz, Perry and Berch.

The preferred detergent-active compounds that can be used are soaps and synthetic non-soap anionic and nonionic compounds.

Anionic surfactants are well-known to those skilled in the art. Examples include alkylbenzene sulphonates, particularly linear alkylbenzene sulphonates having an alkyl chain length of C₈-C₁₅; primary and secondary alkylsulphates, particularly C₈-C₁₅ primary alkyl sulphates; alkyl ether sulphates; olefin sulphonates; alkyl xylene sulphonates; dialkyl sulphosuccinates; and fatty acid ester sulphonates. Sodium salts are generally preferred.

Nonionic surfactants that may be used include the primary and secondary alcohol ethoxylates, especially the C₈-C₂₀ aliphatic alcohols ethoxylated with an average of from 1 to 20 moles of ethylene oxide per mole of alcohol, and more especially the C₁₀-C₁₅ primary and secondary aliphatic alcohols ethoxylated with an average of from 1 to 10 moles of ethylene oxide per mole of alcohol.
Non-ethoxylated nonionic surfactants include alkylpolyglycosides, glycerol monoethers, and polyhydroxyamides (glucamide).

The choice of detergent-active compound (surfactant), and the amount present, will depend on the intended use of the detergent composition. For example, for machine dishwashing a relatively low level of a low-foaming nonionic surfactant is generally preferred.

In fabric washing compositions, different surfactant systems may be chosen, as is well known to the skilled formulator, for handwashing products and for products intended for use in different types of washing machine.

The total amount of surfactant present will also depend on the intended end use and may be as low as 0.5 wt%, for example, in a machine dishwashing composition, or as high as 60 wt%, for example, in a composition for washing fabrics by hand. In compositions for machine washing of fabrics, an amount of from 5 to 40 wt% is generally appropriate.

Detergent compositions suitable for use in most automatic fabric washing machines generally contain anionic non-soap surfactant, or nonionic surfactant, or combinations of the two in any ratio, optionally together with soap.

Laundry detergent compositions of the invention will generally also contain one or more detergency builders. The total amount of detergency builder in the compositions will suitably range from 5 to 80 wt%, preferably from 10 to 60 wt%.

Inorganic builders that may be present include sodium carbonate, if desired in combination with a crystallisation seed for calcium carbonate, as disclosed in GB 1 437 950 (Unilever); crystalline and amorphous aluminosilicates, amorphous aluminosilicates and mixed crystalline/amorphous aluminosilicates; layered silicates as disclosed in EP 164 514B (Hoechst); and inorganic phosphates, for example, sodium orthophosphate, pyrophosphate and tripolyphosphate.

Detergent compositions of the invention may contain an alkali metal, preferably sodium, aluminosilicate builder. Sodium aluminosilicates may generally be incorporated in amounts of from 10 to 70% by weight (anhydrous basis), preferably from 25 to 50 wt%.

The preferred crystalline sodium aluminosilicates of this type are the well-known commercially available zeolites A and X, and mixtures thereof; or maximum aluminium zeolite P (zeolite MAP) as described and claimed in EP 384 070B (Unilever).

Organic builders that may be present include polycarboxylate polymers such as polyacrylates, acrylic/maleic copolymers, and acrylic phosphinates; monomeric polycarboxylates such as citrates, gluconates, oxydisuccinates, glycerol mono-, di- and trisuccinates, carboxymethyloxysuccinates, carboxymethyloxymalonates, dipicolinates, hydroxyethyliminodiacetates, alkyl- and alkenylmalonates and succinates; and sulphonated fatty acid salts.

Especially preferred organic builders are citrates, suitably used in amounts of from 5 to 30 wt%, preferably from 10 to 25 wt%; and acrylic polymers, more especially acrylic/maleic copolymers, suitably used in amounts of from 0.5 to 15 wt%, preferably from 1 to 10 wt%.

Detergent compositions according to the invention may also suitably contain a bleach system. Machine dishwashing compositions may suitably contain a chlorine bleach system, while fabric washing compositions may more desirably contain peroxy bleach compounds, for example, inorganic persalts or organic peroxyacids, capable of yielding hydrogen peroxide in aqueous solution.

Suitable peroxy bleach compounds include organic peroxides such as urea peroxide, and inorganic persalts such as the alkali metal perborates, percarbonates, perphosphates, persilicates and persulphates. Preferred inorganic persalts are sodium perborate monohydrate and tetrahydrate, and sodium percarbonate. The peroxy bleach compound is suitably present in an amount of from 5 to 35 wt%, preferably from 10 to 25 wt%.

The peroxy bleach compound may be used in conjunction with a bleach activator (bleach precursor) to improve bleaching action at low wash temperatures. The bleach precursor is suitably present in an amount of from 1 to 8 wt%, preferably from 2 to 5 wt%. Preferred bleach precursors are peroxycarboxylic acid precursors, more especially peracetic acid precursors and peroxybenzoic acid precursors; and peroxycarbonic acid precursors. An especially preferred bleach precursor suitable for use in the present invention is N,N,N',N'-tetracetyl ethylenediamine (TAED).

A bleach stabiliser (heavy metal sequestrant) may also be present. Suitable bleach stabilisers include ethylenediamine tetraacetate (EDTA) and the polyphosphonates such as Dequest (Trade Mark), EDTMP.

Other materials that may be present in laundry detergent compositions of the invention include sodium carbonate; sodium silicate; antiredeposition agents such as cellulosic polymers; soil release polymers; fluorescers; inorganic salts such as sodium sulphate; lather control agents or lather boosters as appropriate; enzymes (proteases, lipases, cellulases, amylases); dyes; coloured speckles; perfumes; foam controllers; and fabric softening compounds.

While the above description applies generally to laundry detergent compositions, it is especially applicable to powders.

### Liquid laundry detergent compositions

Liquid laundry detergents according to the invention may be built or unbuilt and may be aqueous or nonaqueous. The compositions generally comprise about 5% to 70% by weight of a detergent active material and optionally from 0% to 50% of a builder. The liquid detergent compositions of the invention may further comprise an amount of electrolyte (defined as any water-soluble salt) whose quantity depends on whether or not the composition is structured. By structured is meant the formation of a lamellar phase sufficient to endow solid suspending capability.

The liquid detergent composition generally further comprises enzymes such as proteases, lipases, amylases and cellulases. Stabilisers or stabiliser systems may be used in conjunction with the enzymes. The compositions may also contain various optional ingredients, for example, phase regulants, polyacids, suds regulants, opacifiers, antioxidants, bactericides, dyes, perfumes, and brighteners.

Liquid detergent compositions may be either aqueous or non-aqueous. Non-aqueous liquid compositions contain not more than about 5 wt% water.

Aqueous liquid detergent compositions of the invention may contain deflocculating polymers such as described in US 5 071 586 (Kaiserman et al).

### Preferred detergent compositions

Preferred laundry detergents according to the invention comprise:
(a) from 5 to 70 wt% of an organic surfactant system which comprises a compound of the invention optionally in admixture with one or more anionic, nonionic, zwitterionic, amphoteric or cationic cosurfactants,
(b) optionally from 10 to 80 wt% of one or more detergency builders,
(c) optionally other detergent components to 100 wt%.

### Personal product compositions

Personal product compositions containing compounds of the invention may be, for example, soap bar compositions, facial or body cleansing compositions, shampoos for hair or body, hair conditioner compositions, cosmetic compositions or underarm deodorant/antiperspirant compositions.

Typical toilet soap bar compositions comprise fatty acid soaps used in composition with a non-soap detergent and free fatty acid. Mildness improving salts, such as alkali metal isethionate, are also typically added. In addition other ingredients, such as germicides, perfumes, colorants, pigments, suds-boosting salts and anti-mushing agents may also be added. Other optional ingredients which may be present in soap bar compositions are moisturisers, water-soluble polymers, sequestering agents and emollients.

The surfactant of the invention may typically comprise 0.01 to 45% by weight of the composition.

Typical face or body cleansing compositions will comprise a fatty acid soap together with a non-soap surfactant, preferably a mild synthetic surfactant. Cleansing compositions will also generally include a moisturiser or emollient and polymeric skin feel and mildness aids, and may further optionally include thickener, conditioners, water soluble polymers, dyes, hydrotropes, brighteners, perfumes and germicides.

### EXAMPLES

The invention is further illustrated by the following Examples, in which all parts and percentages are by weight unless otherwise stated.

### EXAMPLES 1 and 2: PREPARATION OF AMIDE SURFACTANTS

The reagents used were as follows:
D-glucamine (ex Janssen Chimica);
dodecyl aldehyde (ex Aldrich);
tetradecyl aldehyde (ex Aldrich);
acetic anhydride (ex Fisher Scientific).

### Example 1 - Preparation of C₁₂ oxazolidine acetamide

In a 2-neck 2-litre round bottom flask were added D-glucamine (30 g, 0.166 moles) and 1.5 litres of anhydrous methyl alcohol. The reaction was stirred (via a magnetic stir bar) and refluxed using an oil bath. After vigorous refluxing, the solution became clear and the reaction was cooled to room temperature. Dodecyl aldehyde (33.05 g, 0.179 moles) and 1.1 g of anhydrous p-toluenesulphonic acid was added to the reaction mixture. The reaction mixture was refluxed for 24 hours and then cooled to 10°C using an ice bath. Addition of acetic anhydride (17.80 g, 0.174 moles) soon followed and the reaction was allowed to run at room temperature for an additional 12 hours.

The reaction was worked up by removal of the methanol solvent. Recrystallisation in acetone gave approximately 65 g of crude material. Further analysis indicated that the this material contained two diasteromers as analysed by NMR and mass spectrometry. The two pure diasteromers were isolated by chromatography under the following conditions:

Column chromatography was carried out on a column packed with C₁₈- (Regis) Bodman Biochrom 1040 using ODS FEC PQ packing material. The solvent used was 55:45 CH₃CN:H₂O. After separation the purity was analysed as follows on an HPLC.

A column having the dimension 5 µm x 15 cm x 4.6 cm was packed with Spherisorb hexyl using the mobile phase containing the following solvent: 30%/30%/40% CH₃OH/CH₃CN/H₂O (volume percent). 14 g/l NaClO₄ (sodium perchlorate) was used in the solvent system and the column temperature was 35°C.

One of the diastereomers showed the following characteristics:
¹³C NMR in CD₃OD: 14.48, 21.96, 23.22, 23.75, 24.36, 24.44, 30.50, 30.65, 30.74, 30.81, 33.09, 34.20, 64.73, 71.95, 72.31, 72.36, 72.46, 72.69, 80.22, 80.56, 90.58, 90.81, 170.10, 170.73.

### Example 2 - Preparation of C₁₄ tetrahydrooxazine myristamide

A solution of glucamine (20 g) and 500 ml of methanol was heated for 2 hours under reflux. The solution was cooled to room temperature and then 37% formaldehyde solution (10.8 ml) was added followed by addition of p-toluenesulphonic acid (2 g). The reaction mixture was stirred at room temperature overnight. The solvent was removed by azeotropic distillation with toluene. The crude tetrahydrooxazine was not purified.

To a solution of tetrahydrooxazine of glucamine (22 g, 0.113 moles) in dry dimethylformamide (50 ml) was added dry pyrridine (25 ml). The solution was cooled to 0°C using an ice bath. Myristoyl chloride (35.59 ml, 0.13 moles) was added portionwise over a 15-minute period. The reaction was stirred for 3 hours at 0°C and then at room temperature overnight. Ice was added to the reaction mixture which was then extracted with methylene chloride (3 x 200 ml) and dried over anhydrous sodium sulphate. Filtering the sodium sulphate and removal of the solvent gave the crude product which was further recrystallised from acetonitrile:water (8:2). The pure product was analysed by NMR, IR, and mass spectrometry.

### EXAMPLES 3 to 6: DETERGENCY CHARACTERISTICS

### Example 3: Critical micelle concentration (CMC)

The CMC is defined as the concentration of a surfactant at which it begins to form micelles in solution. Specifically, materials that contain both a hydrophobic group and a hydrophilic group (such as surfactants) will tend to distort the structure of the solvent (ie water) they are in and therefore increase the free energy of the system. They therefore concentrate at the surface, where, by orienting so that their hydrophobic groups are directed away from the solvent, the free energy of the solution is minimized. Another means of minimising the free energy can be achieved by the aggregation of these surface-active molecules into clusters or micelles with their hydrophobic groups directed toward the interior of the cluster and their hydrophilic groups directed toward the solvent.

The value of the CMC is determined by surface tension measurements using the Wilhemy plate method. While not wishing to be bound by theory, it is believed that a low CMC is a measure of surface activity (ie lower CMC of one surfactant versus another indicates the surfactant with lower CMC is more surface active). In this regard, it is believed that lower CMC signifies that lesser amounts of a surfactant are required to provide the same surfactancy benefits as a surfactant with higher CMC.

The critical micelle concentration of the C₁₂ oxazolidine acetamide of Example 1 at 25°C was measured using the DeNouy ring method. A Lauda TE-1C Tensiometer was used for the experiment. For comparison, the CMC for a heptaethoxylated dodecyl alcohol (typical nonionic surfactant was determined.

Thus, it can be seen that CMC values for these oxazolidines and commercially available ethoxylated nonionic surfactants are comparable.

| | | |
|---|---|---|
| Compound of Example 1 | (25°C) | 1.78 x 10⁻⁵ M |
| C₁₂ alcohol 7EO | (40°C) | 7.3 x 10⁻⁵ M |

### Example 4: Krafft Point

The Krafft temperatures of the surfactants of the invention were measured using 0.10% solutions of surfactants in 100 ml glass jars. 0.050 g of the surfactant was added to 50 g of water, the mixture was stirred and slowly heated by using a water bath. The Krafft point temperature of the surfactant was at the temperature where all the solid surfactant went into solution. The Krafft temperatures are summarised below.

| Surfactant | Krafft Temperature |
|---|---|
| C₁₂ Oxazolidine Acetamide | 36-37°C |
| C₁₄ Oxazolidine Acetamide | 39-40°C |
| C₁₂ N-methyl glucamide (comparative) | 45.3°C |

Once again, those values are comparable to other well known commercially available surfactants indicating that the surfactants of the invention are a viable alternative to those other surfactants.

### Example 5: foam height

Foam heights were measured using the Ross-Miles method and apparatus (see Ross J and Miles G, Am Soc for Testing Materials Method D1173-53, Philadelphia, Pa. (1953); Oil and Soap (1958) 62:1260).

200 ml of a solution of surfactant (0.10% concentration) contained in a pipette of specified dimensions with a 2.9 mm internal diameter opening was allowed to fall 90 cm onto 50 ml of the same solution contained in a glass vessel maintained at various temperatures by means of a water jacket. The height of the foam was read immediately and final foam height was read after a period of 30 minutes although it was generally done after 5 minutes. The measurement was done at room temperature (25°C).

| Compound | Foam Height (Initial) | Foam Height (Final) |
|---|---|---|
| C₁₂ oxazolidine acetamide | 154.185 mm | 134.185 mm |

These values indicate that the surfactant is a good foamer.

### Example 6: detergency on test cloths

The detergency of the oxazolidine acetamide surfactants was measured on WFK-30K test cloths. These are polyester cloths soiled with a synthetic pigment/sebum mixture, the synthetic pigment consisting of 86% kaolinite, 8% flame soot 101, 4% iron oxide (black) and 2% iron oxide (yellow), applied at a concentration of 7.5 g/l from a solution also containing 20 g/l of synthetic sebum consisting of 18.0% free fatty acids, 32.8% beef tallow, 3.6% fatty acid triglycerides, 18.3% lanolin, 3.7% cholesterol, 12.0% hydrocarbon mixture, 11.6% cutina. These mixed solutions are sprayed onto the fabrics in an amount of 150 ml per m of fabric, correlating to an application of 1 g/m² of the pigment mixture and 6 (respectively 3) g/m² of synthetic sebum.

The WFK-30D cloths were cut into swatches of 4" x 3" dimensions and their initial reflectometer values recorded (front and back).

These cloths were washed (four swatches per pot) under the conditions shown below:
- Apparatus:: Terg-O-Tometer UR 7227
- Wash time:: 15 minutes
- Agitation:: 100 rpm
- Wash liquid volume:: 1000 ml
- Dosage:: approximately 1.0 g/l
- Total surfactant level:: 0.22 g/l
- Zeolite 4A:: 0.45 g/l
- Sodium carbonate:: 0.30 g/l
- pH:: 10.0
- Water hardness: 120.0 ppm as 2:1 Ca:Mg
- Temperature: 40 and 25°C

The detergency was measured by change in reflectance values (delta R) between the soiled cloth and the cloth after wash in the tergotometer. Reflectances were measured using a BYK Gardner Cologuard 2000/05 Reflectometer.

Comparative measurements were also carried out using a commercially available ethoxylated alcohol nonionic surfactant, Neodol (Trade Mark) 25-7 ex Shell, a C₁₂₋₁₅ alcohol with an average degree of ethoxylation of 7.

The results are shown below.

| At 25°C | |
|---|---|
| C₁₂ oxazolidine acetamide | 24.75 |
| C₁₂₋₁₅ alcohol 7EO | 21.53 |

| At 40°C | |
|---|---|
| C₁₂ oxazolidine acetamide | 21.50 |
| C₁₂₋₁₅ alcohol 7EO | 18.09 |

Based on these results, the C₁₂ oxazolidine acetamide is comparable or better than Neodol 25-7 at 25 to 40°C under these conditions.

### EXAMPLE 7: PERSONAL WASHING COMPOSITION

A toilet bar soap composition containing a surfactant according to the invention is as follows:

| Ingredients | % by Weight |
|---|---|
| C₈₋₂₄ fatty acid soap | 30-95% |
| Surfactant of invention | 0-45% |
| Alkyl sulphate | 0-5% |
| Moisturiser, eg sorbitol, glycerol, water-soluble polymer (eg cellulase or polyacrylate) | 0.1-10% |
| Sequestering agents (eg citrate) | 0.1-0.5% |
| Dyestuff | <0.1% |
| Optical brighteners | <0.1% |
| Whitening agents | 0.1-0.4% |
| Fragrance | 0.1-2.0% |
| Water | Balance |

### EXAMPLE 8: FACIAL OR BODY CLEANSER

A facial or body cleansing composition containing a surfactant according to the invention is as follows:

| Ingredients | % by Weight |
|---|---|
| C₈₋₂₄ fatty acid salt (eg triethanolamine salt) | 1-45% |
| Surfactant of invention | 10-75% |
| Alkyl sulphate | 0-20% |
| Cosurfactants (eg cocoamidobetaine) | 1-15% |
| Moisturiser (eg sorbitol) | 0.1-15% |
| Refattying alcohol | 0.1-5% |
| Water soluble polymer | 0-10% |
| Thickener | 0-15% |
| Conditioner (eg quaternised cellulose) | 0-0.5% |
| Sequestering agents (eg citrate) | 0.1-0.4% |
| Dyestuff | <0.1% |
| Optical brighteners | <0.1% |
| Whitening agents | 0.1-0.4% |
| Fragrance | 0.1-3.0% |
| Preservatives | 0-0.2% |
| Water | Balance |

## Claims

1. A compound having the formula I(a) or I(b): 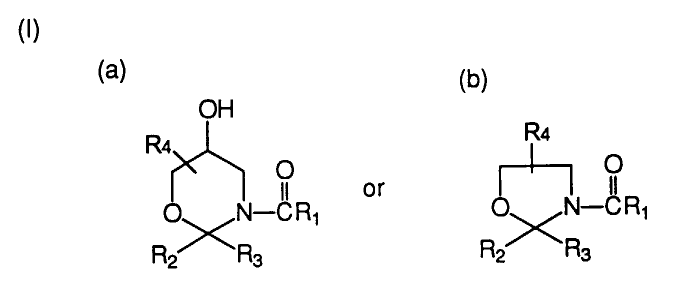
wherein R₁ is a linear or branched alkyl or alkenyl group having from 1 to 50 carbon atoms;
each of R₂ and R₃, which may be the same or different, is hydrogen or a linear or branched alkyl or alkenyl group having from 1 to 50 carbon atoms; and
R₄ represents the residue of a reducing sugar or an amino sugar.

2. A compound as claimed in claim 1, wherein R₄ represents the residue of a sugar selected from glucose, fructose, maltose, lactose, galactose, mannose, xylose, erythritose and glyceraldehyde.

3. A compound as claimed in claim 1, wherein R₄ represents the residue of an amino sugar selected from glucamine and glucosamine.

4. A compound as claimed in claim 1, which is a 6-membered tetrahydrooxazine compound of the formula I(a) wherein R₄ is
-(CHOH)_{n'}-CH₂OH
wherein n' is from 1 to 5.

5. A compound as claimed in claim 4, wherein R₂ = R₃ = hydrogen and R₁ = C₁₁ to C₁₇.

6. A compound as claimed in claim 1, which is a 5-membered oxazolidine compound of the formula I(b) wherein R₄ is
-(CHOH)_{n'}-CH₂OH
wherein n' is 1 to 5.

7. A compound as claimed in claim 6, wherein R₂ = hydrogen, R₁ = CH₃ and R ₃ = C₁₁ to C₁₇.

8. A compound as claimed in claim 1, which is C₁₂ or C₁₄ oxazolidine acetamide.

9. A compound as claimed in claim 1, which is C₁₄ tetrahydrooxazine myristamide.

10. A process for the preparation of a compound as claimed in claim 1, which process comprises:
(i) cyclising an amino sugar by reaction with an aldehyde to form either a 5-membered oxazolidine or 6-membered tetrahydrooxazine,
(ii) amidating the 5-membered oxazolidine or 6-membered tetrahydrooxazine by reaction with an acid anhydride or acyl halide, to form a compound of the formula I.

11. A detergent, cleaning or personal care composition comprising a surfactant system which consists wholly or partially of a compound as claimed in any one of claims 1 to 9.

12. A composition as claimed in claim 11, which contains from 0.01 to 60 wt% of the compound as claimed in any one of claims 1 to 9.

13. A composition as claimed in claim 11, which contains from 0.1 to 45 wt% of the compound as claimed in any one of claims 1 to 9.

14. A composition as claimed in claim 12, which contains from 1 to 20 wt% of the compound as claimed in any one of claims 1 to 9.

15. A composition as claimed in any one of claims 11 to 13, which is a liquid or powder laundry detergent composition comprising:
(a) from 5 to 70 wt% of an organic surfactant system which comprises a compound as claimed in any one of claims 1 to 7 optionally in admixture with one or more anionic, nonionic, zwitterionic, amphoteric or cationic cosurfactants,
(b) optionally from 10 to 80 wt% of one or more detergency builders,
(c) optionally other detergent components to 100 wt%.

16. A composition as claimed in claim 11, which is a personal product composition selected from the group consisting of soap bar compositions, facial or body cleansing compositions, shampoo compositions, conditioner compositions, cosmetic compositions and underarm deodorant/antiperspirant compositions
